# EUROPEAN PATENT SPECIFICATION

(11) **EP 0 675 742 B1**
(45) Date of publication and mention of the grant of the patent: **04.03.1998**
(21) Application number: 94902948.2
(22) Date of filing: 23.12.1993
(51) Int. Cl.: A61M 15/00

(54) **INHALER DEVICES**
INHALATIONSAPPARAT
INHALATEURS

(30) Priority: 24.12.1992 GB 9226901; 24.12.1992 GB 9226918; 24.12.1992 GB 9226951; 24.12.1992 GB 9226969
(43) Date of publication of application: 11.10.1995
(60) Divisional application: 97110944.2
(73) Proprietor: RHONE-POULENC RORER LIMITED, West Malling, Kent ME19 4TA (GB)
(72) Inventor: HOBBS, Michael Anthony, Dagenham, Essex RM10 7XS (GB); CALVERT, John Richard, Dagenham, Essex RM10 7XS (GB); COOK, Robert Stanley, Dagenham, Essex RM10 7XS (GB); TRUNLEY, Roy, Dagenham, Essex RM10 7XS (GB); SIMPKIN, Gordon Thomas, Dagenham, Essex RM10 7XS (GB)
(74) Representative: Caffin, Lee
(86) International application number: GB9302642
(87) International publication number: WO9414491

(56) References cited:
- EP-A- 0 424 790
- EP-A- 0 506 293
- WO-A-82/01470
- WO-A-92/03175
- FR-A- 2 660 550
- GB-A- 2 255 918

## Description

### Field of the Invention

The present invention relates to inhalers for the inhalation of a medicament, usually pulverulent, from a container, for example a hard gelatin capsule, and more specifically to inhalers with magazines containing several capsules to be used one at a time.

### Background of the Invention

Various forms of multi-unit dose inhalers are known and among these are ones in which a main housing contains a blister pack. The blister pack has a plurality of blisters each defining a container having a dosage of medicament and which are burst individually in order for a user to inhale the medicament. The blisters remain joined to a carrier during extraction of the medicament.

Further multi-unit dose inhalers, such as are shown in document WO 82/01470 upon which the preamble of claim 1 is based, include those using capsules which are held in a rotating magazine. One end of a capsule is pulled away from the other to allow access of air to the contents, whereby they can be inhaled. Alternatively the capsules may be pierced to gain access to their contents after which the user may inhale the medicament.

In such an arrangement the extraction of medicament usually occurs as a result of an inhaled airstream passing over a capsule or other container.

It is necessary to provide access to the contents of a capsule or blister pack. This can be done by piercing the container, which may lead to the drawback of needing to provide piercing pins in the inhaler, such as in document WO 92/03175, and which, if an inhaler is handled carelessly, could cause discomfort. Also, if a container is pierced in situ it can lead to fragments of the container being inhaled with the contents.

It is a disadvantage of most known inhalers that not all of the medicament, or even a large proportion of it, is drawn from the capsule, or the separated capsule cap and body portions, or the blister pack.

It is known, for example from document GB-A-1,562,732, that a swirling chamber using a combination of pneumatic action, centrifugal action and impact to extract medicament from a capsule is very effective and it is preferred that the inhaler should be capable of using such actions in such a swirling chamber without over-complicating the device.

Preferably a capsule should be automatically loaded and pierced without loss of contents prior to entry into the medicament chamber.

However, the spent capsules must then be removed in order to allow access for the next unused capsule.

It is a disadvantage of known inhalers that it is often necessary to carry out many manipulative movements and capsule transfers before the medicament can be inhaled. In the case of someone who is unable, especially at the time the medicament is most needed, to perform all these functions these known inhalers can be inconvenient.

Further, it is not often, if ever, clear how many capsules a magazine has still left. Even if it can be seen how many are left, if all the capsules have been used it will still be possible for the user to attempt to inhale (on a used capsule).

### Brief Summary of the Invention

It is an object of the present invention to provide a magazine for use in a multi-unit dose inhaler in which at least some of the above mentioned disadvantages are eliminated.

According to a first aspect of the present invention this object is met by a rotary magazine for use in an inhaler, comprising a plurality of recesses for housing capsules, and means, in a plurality of said recesses, for holding the capsules; characterised
in that said magazine is adapted to receive pierced capsules;
by further comprising means for plugging pierced holes in the capsules, and means to release said capsules one at a time, from said magazine; and
in that said plugging means are provided in a plurality of individual ones of said recesses.

Preferably a capsule should be automatically loaded into the magazine and plugged, without loss of contents prior to entry into the medicament chamber.

It is very useful to know how many capsules or doses are left in a magazine before it needs to be recharged and therefore an indicator may be provided on the magazine.

The magazine can be used with capsules which are pierced before they are loaded into the magazine, or pierced in the magazine.

The magazine should hold a plurality and preferably at least eight such capsules.

The invention also provides an inhaler in combination with such a magazine.

### Brief Description of the Drawings

In order that the present invention may be more readily understood the following description is given, merely by way of example, with reference to the accompanying drawings, in which:
FIGURE 1 is a side elevational view of a first inhaler embodiment for use with a magazine in accordance with the present invention;
FIGURE 2 is a top plan view of the inhaler of Figure 1;
FIGURE 3 is a side sectional view taken along the line 3-3 of Figure 2;
FIGURE 4 is a sectional view taken along the line 4-4 of Figure 3;
FIGURE 5 is a view similar to Figure 3 but showing the inhaler, partway through the operating cycle when the capsule magazine has just been rotated;
FIGURE 6 is a bottom sectional view taken along the line 6-6 of Figure 3;
FIGURE 7 is a side sectional view of a first rotary magazine embodiment in accordance with the present invention for use in the inhaler of Figure 1;
FIGURE 8 is a view of the same section as Figure 7 when the magazine has been rotated to a position where a capsule is ready for use;
FIGURE 9 is a development view of the capsule release cam tracks of the magazine of Figures 7 and 8;
FIGURE 10 is an enlarged view of part of Figure 7;
FIGURE 11a is an end elevational view of the magazine of Figure 7;
FIGURE 11b is a side elevational view of the magazine of Figure 7;
FIGURE 12 is a side sectional view of a second rotary magazine embodiment according to the present invention for use in the inhaler of Figure 1;
FIGURE 13 is a view of the same section as Figure 12 when the magazine has been rotated to a position where a capsule is ready for use;
FIGURE 14 is an elevational view of a daisy wheel used in the magazine of Figures 12 and 13;
FIGURE 15 is a side elevational view of a second embodiment of inhaler;
FIGURE 16 is a top plan view of the inhaler shown in Figure 15;
FIGURE 17 is a partly sectioned side view, corresponding to Figure 15, and showing the second embodiment of inhaler;
FIGURE 18 is a partially sectioned top view of the inhaler of Figures 15 to 17;
FIGURE 19 is a sectional view of the inhaler of Figures 15 to 18, taken on the line 19-19 of Figure 16;
FIGURE 20 is a sectional view taken on the line 20-20 of Figure 15;
FIGURE 21 is a partial elevation of the magazine housing when viewed from the rear of Figure 15;
FIGURE 22 is a partial elevation of the magazine housing when viewed along the direction of arrow 22 in Figure 15;
FIGURE 23 is a longitudinal transverse section of a third embodiment of the capsule magazine in accordance with the present invention;
FIGURE 24 is a partly sectioned elevational detail of the left hand end of the magazine of Figure 23;
FIGURE 25 is an end view of the magazine shown in Figures 23 and 24.
FIGURE 26 is a longitudinal section taken through a fourth embodiment of the capsule magazine in accordance with the present invention;
FIGURE 27 is a sectional view of the magazine of Figure 26 but viewed along the direction of arrow 27 of Figure 26; and
FIGURE 28 is a view of the magazine of Figure 26 when viewed from the left hand side and with the end cap 704 removed.

### Detailed Description of Preferred Embodiments

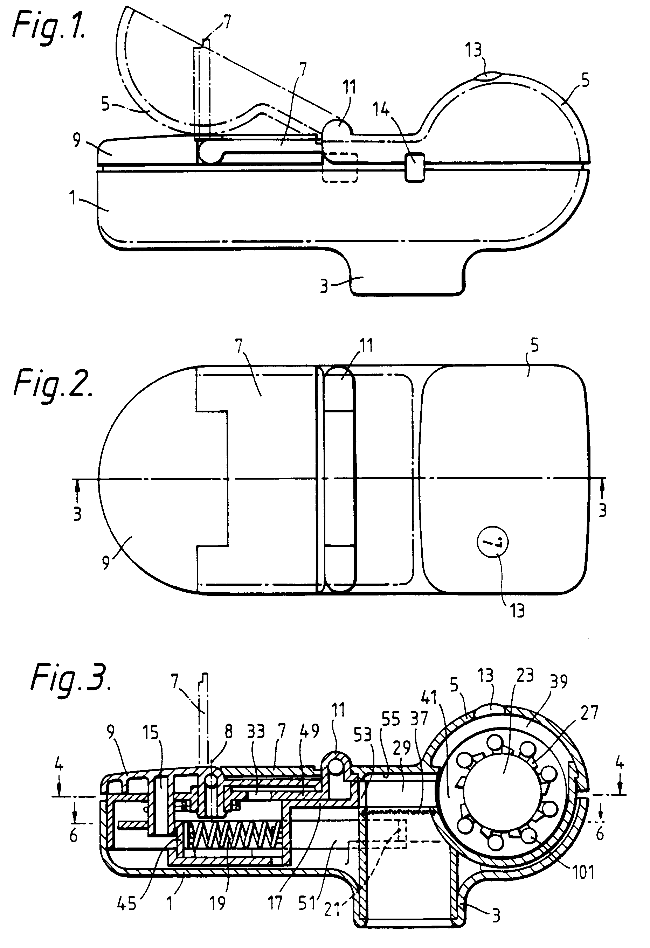

Referring now to the drawings, wherein the numerals indicate like elements throughout, a first embodiment of an inhaler for use with the present invention will be described with reference to Figures 1, 2, 3, 4, 5 and 6.

Referring now to the drawings, a first embodiment of an inhaler according to the present invention will be described with reference to Figures 1 to 6. The inhalers of the first embodiment are for using magazines with capsules which do not need to be pierced by pins provided in the inhaler at the moment of preparation but are already pierced in the magazines.

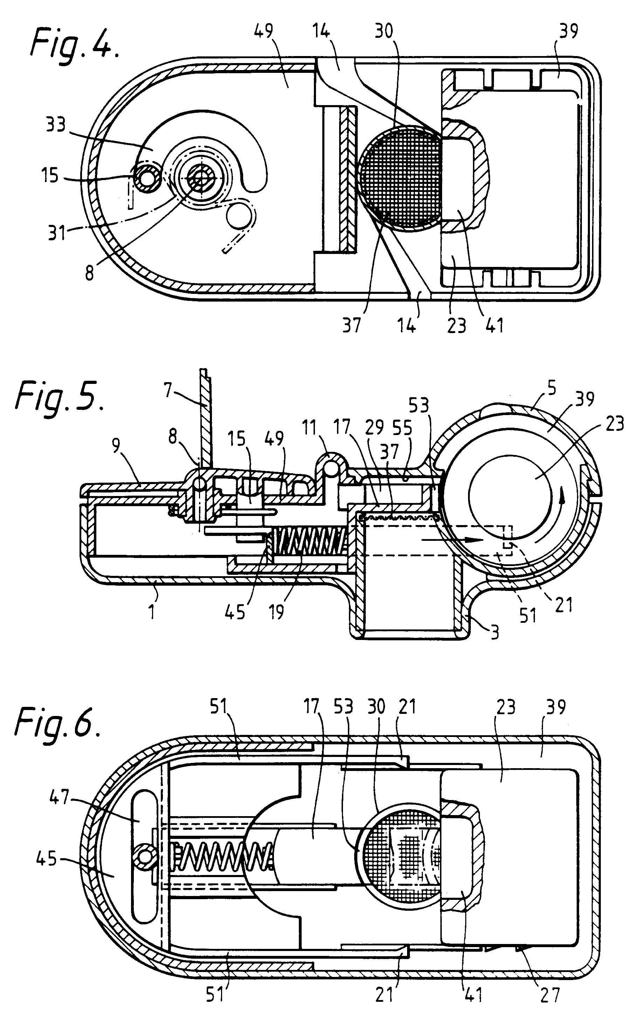

Figure 1 shows a side view of an inhaler according to a first embodiment of the present invention. From Figures 1 to 3 it can be seen that there is a main body (1) from one face of which protrudes a mouthpiece nozzle (3) through which medicament held in capsules is drawn out and delivered to a user in a capsule-emptying phase, taking place in a swirling chamber (29) in the main body. On the other face of the main body from the mouthpiece nozzle (3) is situated the magazine chamber cover (5) rotatable about a pivot (11). When the magazine chamber cover (5) is opened, a used rotary magazine (23) of spent medicament capsules can be removed from the magazine chamber (39) and/or a new one inserted. Also accessible from the outside there is provided a key (7) which is pivoted at one end about a first axis and may be lifted up to the position shown by the dotted lines in Figure 1. The key (7) is further pivoted to rotate about a vertical second axis (8). Turning this key (7) about the vertical axis (8) operates the mechanism of the inhaler to eject a used capsule from the swirling chamber (29) shown in Figure 6, and to replace it with an unused capsule from the magazine for the operator to use.

There are two air inlets (14) (Figure 1) on the sides of the main body (1). When the user inhales through the mouthpiece nozzle (3) air is drawn in through these inlets (14) into the swirling chamber (29) where it picks up the contents of a capsule and exhausts them through a mesh (37) and the nozzle (3).

The operation of an embodiment, whereby a capsule which has been used and is left in the swirling chamber (29) is removed and replaced by an unused capsule from the rotary magazine (23), will be described with reference to Figures 3, 4, 5 and 6.

When the operator wishes to use the inhaler he lifts the key (7) to its vertical position and rotates it about the vertical axis (8) in a clockwise direction.

There is also provided a cover plate (9) on the outside of the inhaler, which is flush with the key (7) in its horizontal position. In rotating the key (7) the cover plate (9), pivotally attached to the key (7), is also caused to rotate. Formed as part of, or attached to, the cover plate (9) is a peg (15) which protrudes into the inhaler.

The peg 15 is slotted through an arcuate track (33) formed in an inner frame (49) situated within the main body (1) at an end remote from the magazine chamber (39), and is caused to travel along the arcuate track (33) against the resistance of a spring (31), as the key (7) is turned. The peg (15) also passes through a rectilinear track (47) as can be seen from Figure 6. The rectilinear track (47) is formed in the flat rear part of a movable slide plate (45) situated within the main body. As the peg (15) is rotated around the arcuate track (33) it passes back and forth along the rectilinear track (47) and moves the slide plate (45) forwards, that is to the right in Figures 3 to 6.

As part of, or attached to, the slide plate (45) are driven arms (51) for rotating the rotary magazine (23). Also attached to the slide plate (45), through an optional compression spring (19), is a movable capsule ejector plate (17). The ejector plate (17) is movable across the width of the swirling chamber (29).

As can be seen from Figure 5, when the key (7) and peg (15) are rotated about the vertical axis (8) the slide plate (45), the driven arms (51) (shown in dotted lines in Figure 5) and the ejector plate (17) are advanced. Before the slide plate (45) reaches the position shown in Figure 5 the ejector plate (17) reaches the limit of its possible movement and is prevented from travelling any further. From this point the peg (15) may only travel further along the arcuate track (33) against the resistance of the compression spring (19) as it is compressed. By this further movement the driven arms (51) advance further whilst the ejector plate (17) is stationary.

The forward motion of the driven arms (51) causes the rotary magazine (23) to index by rotating in an anti-clockwise direction, as seen in the orientation of Figure 5, as ratchet pieces (27), formed on both sides of the rotary magazine (23), are caught by protrusions (21) on the driven arms. Once the peg (15) has reached the limit of its movement along the arcuate track and is released, the two springs (19) and (31) rotate it back to the other end of the arcuate track. At the same time the slide plate (45), with the arms (51), and the ejector plate (17) return to their original positions. On the retraction of the driven arms (51) the rotary magazine (23) does not rotate back in a clockwise direction but stays where it has been advanced to by the protrusions (21). Either the magazine itself or the magazine in combination with the magazine chamber (39) is provided with means (not shown) by which this is achieved. Further, means could be used to ensure that a magazine is rotated neither more nor less than the amount necessary for the magazine to feed a new capsule to the emptying or swirling chamber.

During its movement the ejector plate (17) is advanced into the swirling chamber (29) by the rotation of the key (7) about the vertical axis (8). The ejector plate (17) has an end tab (53) seen in Figures 3, 5 and 6. When the ejector plate (17) is in its rest position the end tab (53) forms part of the chamber wall (30) of the swirling chamber (29). Whilst the end tab (53) may be flush with the wall when at rest, it may instead protrude from, or form a recess in, the wall in order to provide irregular surfaces on which, during the inhalation of a medicament from a capsule as described later, a capsule may sustain further impacts. It is preferred that no gaps should be found around the edges of one end tab (53) as it sits in its rest position, so that during inhalation no air may leak past it into the swirling chamber (29) and thereby reduce the swirling effect of the air entering through inlets (14). Also no powder from capsules may work its way into the mechanism and thereby cause a need for more regular cleaning. Preferably a seal is formed between the chamber wall (30) and the edges of the end tab (53). A capsule is trapped in the swirling chamber by a mesh (37) below it, a top wall (55) above it and the peripheral side wall (30).

As the ejector plate (17) with the end tab (53) is advanced by the turning of the key (7) it crosses the diameter of the swirling chamber (29) and pushes any capsule in the swirling chamber (29) towards a chordal recess (41) formed opposite to the end tab (53) and which is part of the rotary magazine (23) and from which, in most cases, the capsule entered the swirling chamber (29). The end tab (53) is arcuate in shape whereby no capsule should be longitudinally trapped between the end tab (53) and the back wall of the recess (41), but instead an end of the capsule slides along the tab (53) and enters the recess (41) along its length. In this fashion, used capsules are removed from the swirling chamber (29) with no need for them to be removed by hand and no ejection of them into the environment. This could otherwise both pollute the environment and present a hazard.

The end tab (53) advances until it has reached its movement limit position, which is defined to be just before it reaches the rotary magazine (23), so that it does not inhibit rotation of that magazine. Any capsule in the swirling chamber (29) would, by the time the end tab has reached its limit position, have been forced into the chordal recess (41) provided at the side of the swirling chamber by the rotary magazine. Further, because the end tab (53) is maintained at its limit position as the magazine (23) is rotated, the capsule is kept by the end tab (53) in its recess (41) as long as is necessary to prevent its escape, that is until after indexing rotation. Apart from at the use position, when a recess (41) in the rotary magazine (23) forms a recess in the swirling chamber (29), the capsules should not be released from the magazine. Once a rotary magazine (23) has been rotated, and the key (7) is released or turned back to its original position, the slide plate (45) also returns towards its original position causing the arms (51) and ejector plate (17) to do so too.

While, according to the drawings, the key (7) is to be rotated about its vertical axis (8) in a clockwise direction, there is no reason why the inhaler could not be manufactured for the key to be rotated in an anti-clockwise direction for use by left-handed people, or for use even in either direction. Further, although the peg (15) is shown to travel back and forth around a semi-circular arcuate track (33), it could travel 360° around a circular, or possibly elliptical track in the course of a single cycle.

On the other hand a rotating key (7) does not have to be the means by which such a device is operated. A rectilinear track and button in the direction of movement of the slide plate (45) and ejector plate could work equally well.

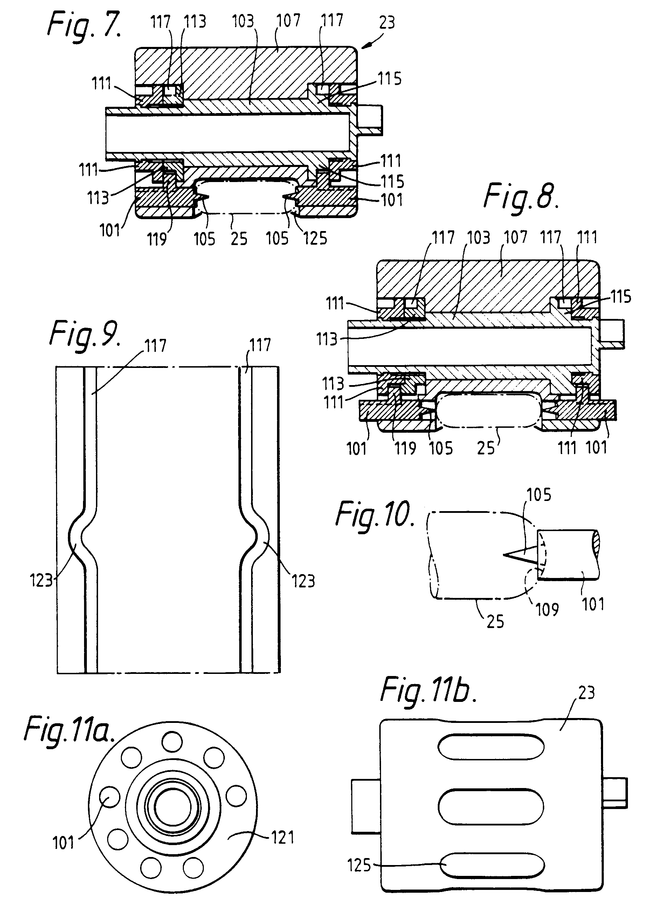

A first type of rotary magazine for use with the first inhaler embodiment of Figures 1 to 6 is now described with reference to Figures 7 to 11.

Figures 7 and 8 show a rotary magazine (23) formed with two main parts, a stator (103) to be held in place when loaded in an inhaler, and a rotor (107). The capsules, eight of them in this embodiment, are held in recesses (125) in the periphery of the rotating part (107), with the longitudinal axes of the capsules and recesses parallel to the axis of rotation of the rotating part (107). In Figures 7 and 9 it can be seen that the capsules (25) are maintained in position by pins (101). In this first magazine the capsules are already pierced during, or prior to, loading in the magazine. The pins (101), as well as maintaining the capsules in position in their recesses, also serve to plug the holes formed in the end of the capsules. These holes can exist either before the capsules are loaded into the rotary magazine (23) or may be made as the rotary magazine (23) is assembled or loaded.

Figure 10 is an enlarged view from Figure 8 and shows the plug, or point (105) of a pin (101) maintained within the ends of a capsule. The sealing walls (109) of the pins are formed so as to maintain a seal around the ends of the capsules, to prevent escapes of powder and, possibly more importantly, to prevent air from entering an unused capsule to contaminate it or to allow its contents to degrade. In this fashion the integrity of the capsules is maintained although they are already pierced.

Additionally it is envisaged that the magazine could be sealed into suitable outer packaging such as a sachet or blister pack to provide further protection against the environment prior to insertion into the inhaler.

Figure 11a shows a blank position (121) in the rotary magazine (23). When a magazine is first inserted into the inhaler it is the blank position (121) which is aligned with the swirling chamber (29). The blank position, which may be an empty recess, prevents the waste of a capsule which, otherwise, would be released immediately upon insertion of the magazine.

Formed within the first type of rotary magazine (23) is a pair of capsule release cam tracks (117), one track being situated towards each end of the magazine. These cam tracks are defined, at the left hand end of Figures 7 and 8, by track pieces (111) and (113) and, at the right hand end, by track pieces (111) and (115). The cam tracks (117) and the track pieces (111,113,115) are stationary and may be formed integrally with or separately from the stator (103), depending upon the method of assembly of the magazine. The locus of the tracks is shown in Figure 9.

The pins (101) are formed with legs (119) as followers to fit into the release cam tracks (117) between the track pieces. As the rotor (107) rotates and the pins (101) rotate with it, so the legs (119) of the pins (101) follow the cam tracks (117). The pins are formed to be axially movable within the rotor (107), that is to the left and right in Figures 7 and 8. Figure 9 shows positions in the cam tracks where there are bulges (123), in both cases towards the nearest axial end of the magazine. As the pin legs (119) follow the tracks, they are caused, at the bulges (123), to move out towards the ends of the magazine. The bulges are situated at the 'use position', i.e. where a recess (125) in a magazine inserted into the inhaler is part of the swirling chamber (29) of the inhaler and a capsule in that recess is to be used. At this point, therefore, the two pins (101) holding that capsule move apart, thereby unplugging their capsule (25) and releasing it. Thus, the capsules are plugged tightly until their recesses (125) reach this 'use position' where they are released. When, after use, they are returned to their recesses by the ejector plate (17) the capsules are kept in their recesses as the magazine is rotated. As a recess (125) is rotated away from the 'use position', the legs of the pins (101) of that recess continue to follow the tracks; they come towards each other again, and re-plug the used capsule. This is useful, not only in preventing capsules from moving about freely, possibly jamming the magazine, but also in preventing any residual powder in spent capsules from escaping to contaminate the inhaler, and causing it to need further cleaning.

In the above described first rotary magazine (23), although the capsules may be pierced before loading into the magazine, the pins are so shaped that they pierce the capsules upon loading of the magazine with the capsules. Alternatively they can be shaped differently so that they merely provide a plug for pre-pierced capsules.

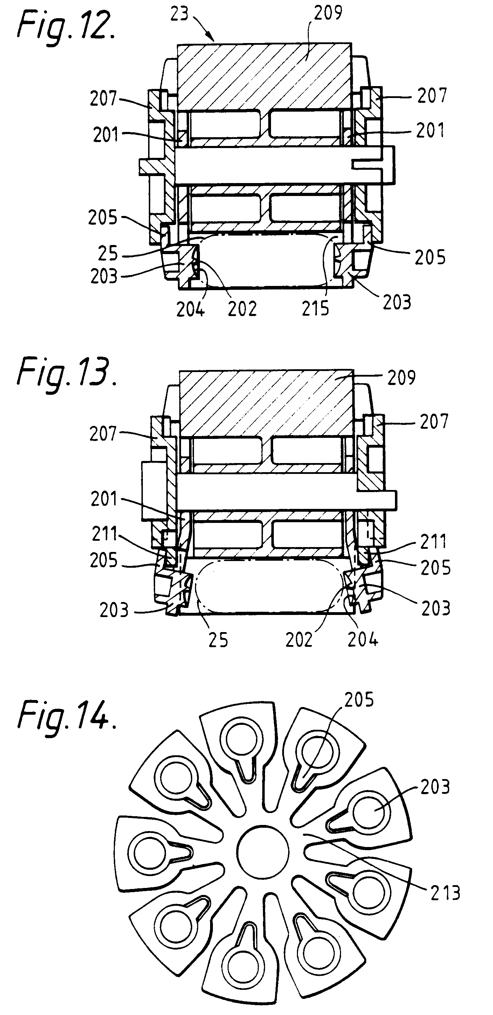

A second type of rotary magazine also for use in the first embodiment of the inhaler is described with reference to Figures 12 to 14. As with the previously described magazine the intention is to deliver a pierced capsule to the 'use position' and the magazine must be inserted correctly to allow this. Again, as with the first described magazine the capsules are to be pierced before a magazine is inserted into the inhaler. In this particular magazine the capsules are pierced, for example by laser, before loading into the magazine, although it is possible to use pins which only pierce the capsules as, or before, they are loaded into the magazine. This design of magazine therefore also maintains a seal on the ends of the capsules until they are required for use and after they are returned.

In this second type of magazine, there is a rotor (209) in the periphery of which are formed the recesses (215) holding the capsules (25), with the longitudinal axes of the capsules and recesses parallel to the axis of rotation of the rotor (209). It is also formed with two end pieces (207) which are stationary and two daisy wheels (201) which rotate with the rotor (209). As with the first type of magazine, the rotor rotates so each capsule is, one after the other, taken to the 'use position' where it may then be used.

The daisy wheels (201) (Figure 14) are formed with a core section (213) and a plugging portion (203), the plugging portions each holding an end of a capsule when a magazine has been loaded. Attached to the plugging portion (203) is a raised portion (205) which is formed to be out of the plane of the rest of the daisy wheel (201) and to leave a gap in the daisy wheel along that length of the main plane running parallel to the extent of the raised portion (205). Both of the end pieces (207) on the stator of the magazine (23) have a projection (211) which exists only where the capsules are to be released at the 'use position'. The projections (211) on both sides of the magazine (23) catch the raised portions (205) of the daisy wheels as they pass. In catching the raised portions the projections move them further apart, as can be seen from Figure 13. Because the daisy wheel is not solid beneath the extent of the raised portions (205), i.e. the raised portions (205) appear as bent tabs leaving an opening in the wheel, no other part of the daisy wheel catches on the projections (211).

Pulling the raised portions away from each other causes the plugging portions (203) of the daisy wheel also to move apart, whereby a capsule held between them is released. Normally there is a residual bias in the daisy wheels (201) to maintain a pressure of the rims (204), surrounding the plugs (202), on the capsules to keep them from falling out and also to maintain a seal on any of those capsules which may be slightly shorter than the average.

Since the projections (211) in the stationary part only exist at the 'use position' the plugging portions (203) of the daisy wheels only release the capsules when they are in the use position. Again, as in the case of the first magazine, when a capsule is returned to its original recess by the ejector plate end tab and the magazine is rotated for the next capsule to be released, the plugging portions (203) reclasp the capsules and prevent them from jamming the mechanism and releasing any residual powder. Also, as with the first type of magazine, magazines of the second type are each provided with a blank position or empty recess.

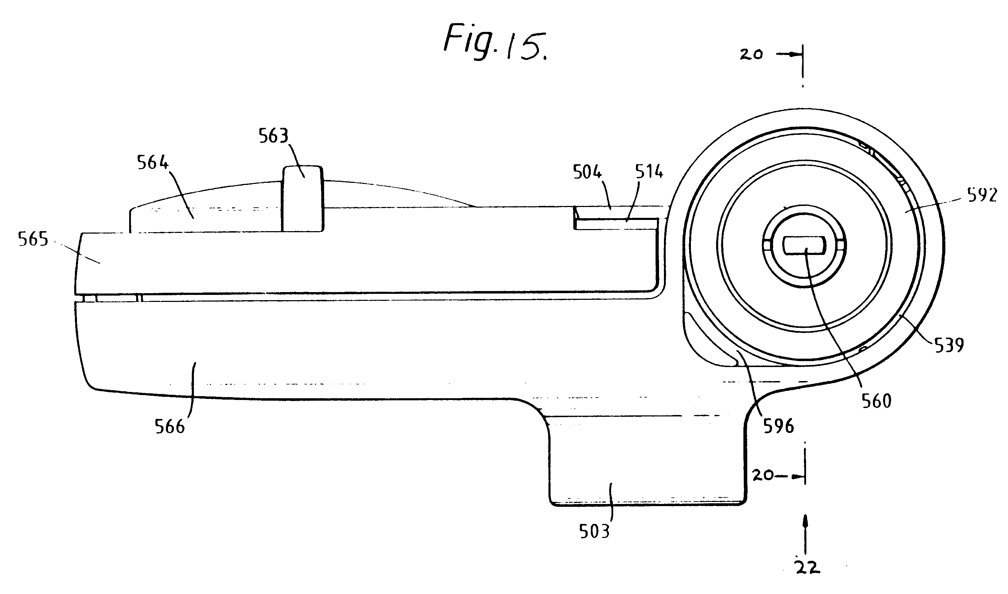

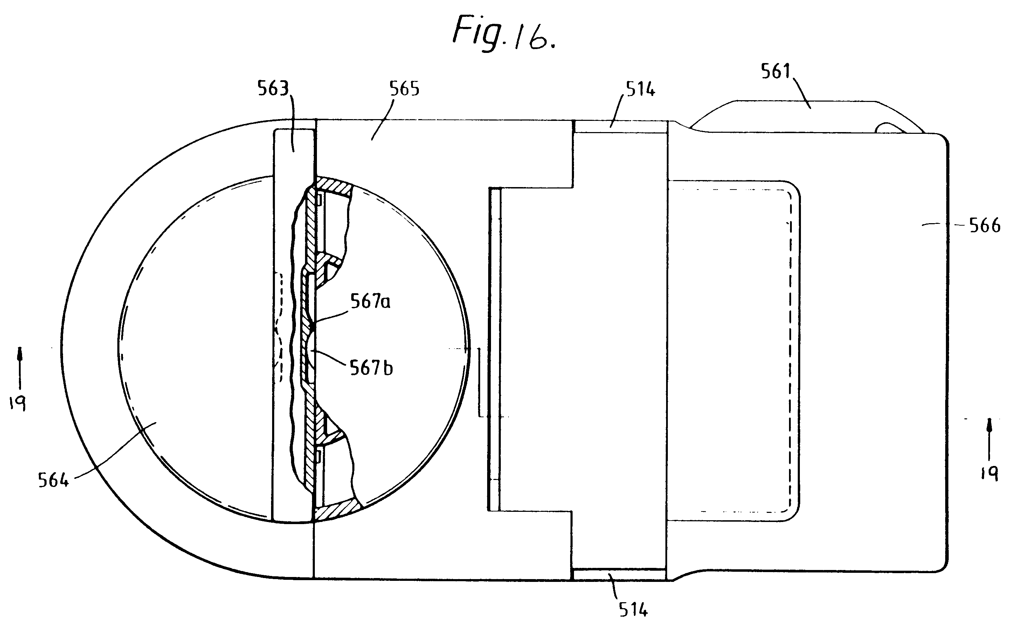

A second embodiment of an inhaler usable with the invention will now be described by reference to Figures 15-21.

There is a front cover (566) from one face of which protrudes a mouthpiece nozzle (503) through which medicament held in capsules is drawn out and delivered to a user. This takes place in a swirling chamber (529) in the rear cover (565) in a capsule-emptying phase.

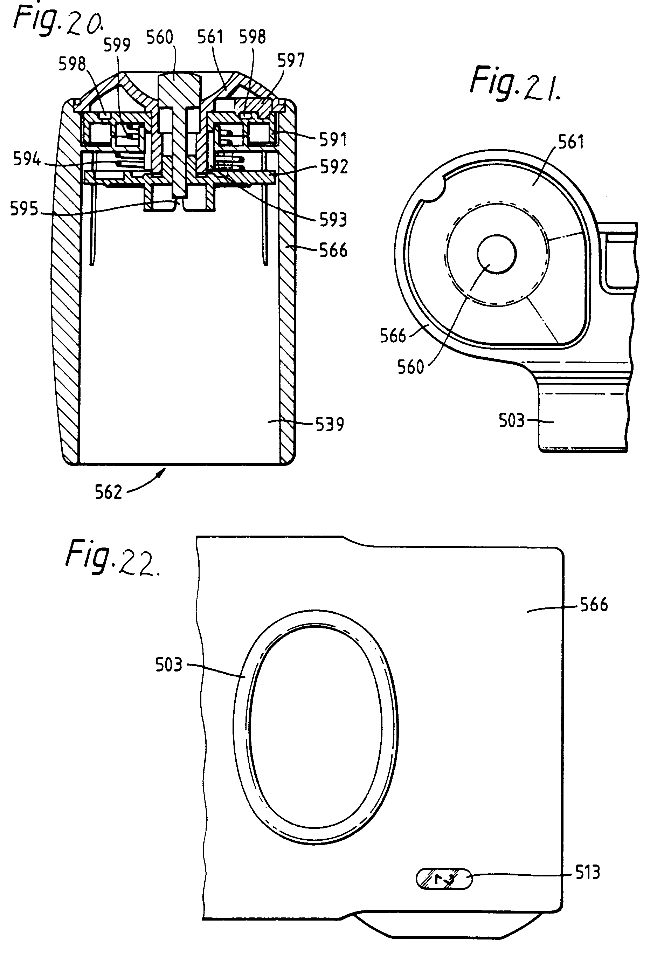

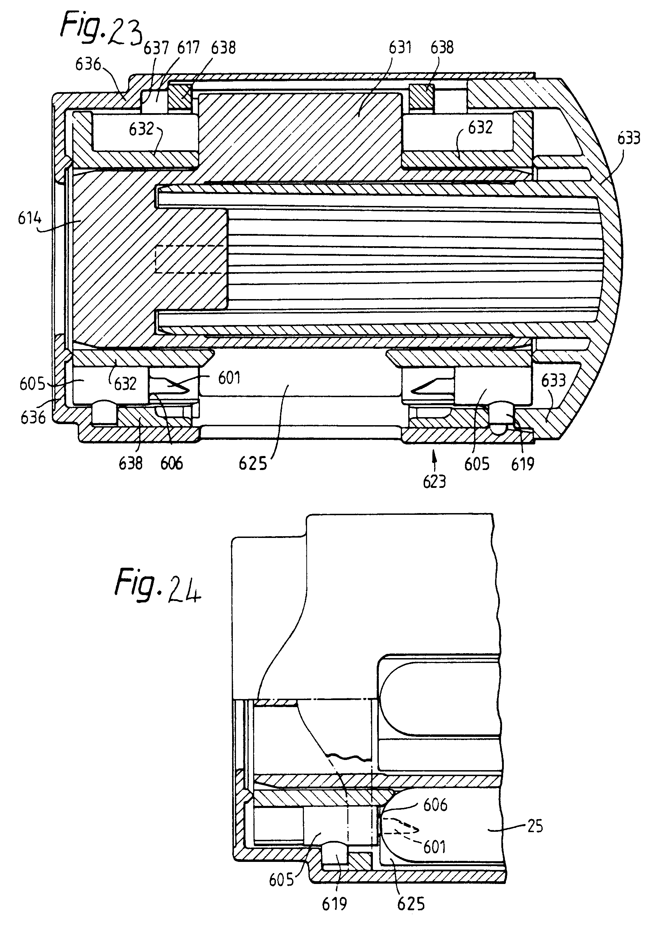

Figure 21 shows a part side view indicating an end cap (561) through which a magazine ejector button (560) protrudes. When the magazine ejector button is depressed the used magazine of spent medicament capsules is ejected through aperture (562) (Fig. 20) in the side of the front cover (566) (Fig. 15). Also accessible from the outside is a slider (563) mounted diametrically of the wheel (564) and which, when moved along its receiving slot diametrically across the wheel (564) by its full extent, may then be used to rotate the wheel through a maximum angle of 180° about a vertical axis (8). Turning this wheel (564) about the vertical axis (8) operates the mechanism of the inhaler to eject a spent capsule from the swirling chamber (529) shown in Figure 28, and to replace it with an unused capsule from the magazine for the operator to use.

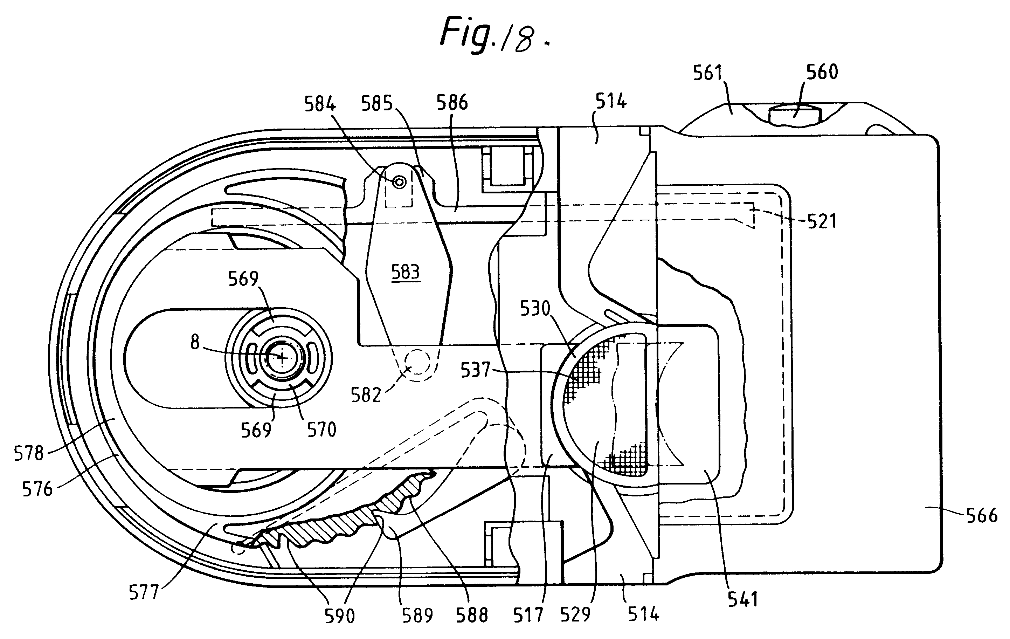

Two air inlet passages (514) (Figure 18) open into the sides of the rear cover (565) as shown in Figure 15. When the user inhales through the mouthpiece nozzle (503) air is drawn through these inlet passages (514) into the swirling chamber (529) where it picks up the contents of a capsule and exhausts them through a mesh (537) and the nozzle (503).

A capsule is trapped in the swirling chamber defined by a mesh (537) below it, by a door (504) above it and by the peripheral side wall (530) (Fig.18). In its open position the door (504) enables the chamber to be accessed for cleaning and in its closed position functions as the further chamber wall constraining the capsule and also completes the air inlet passages (514). The door (504) may be provided with air holes to augment or replace the open ends of the inlet passages (514) so that air may be drawn into the chamber through its sides, rear or both.

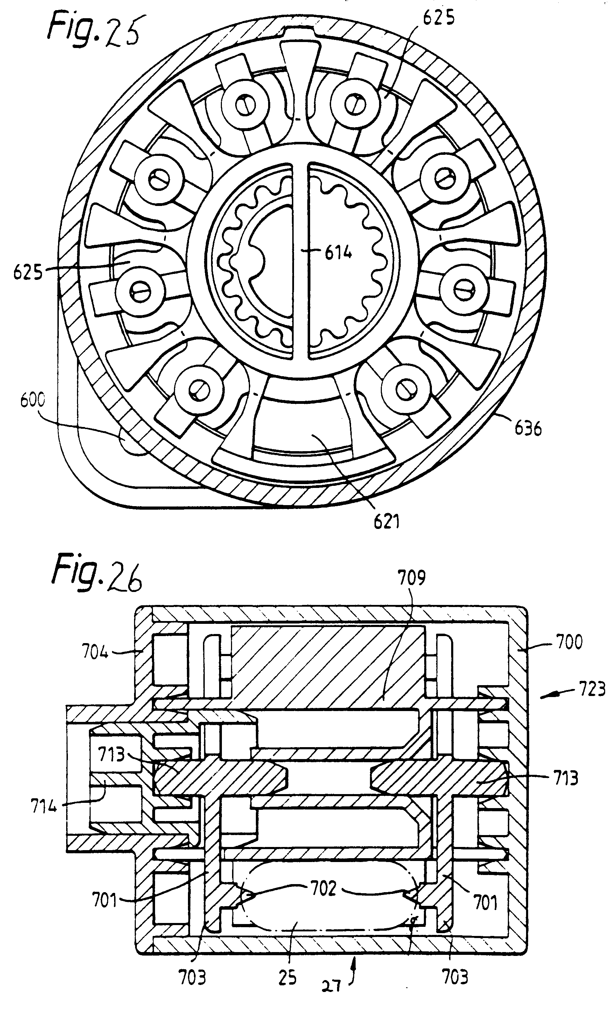

Figure 22 shows a front view of the inhaler of Figure 15. There is a window (513) mounted in the front cover (566) through which can be seen a number representing the number of unused, or alternatively spent, capsules within the loaded rotary magazine.

The operation of this inhaler, whereby a capsule which has been used and is left in the swirling chamber (529) is removed and replaced by an unused capsule from the rotary magazine, will be described with reference to Figures 15-22.

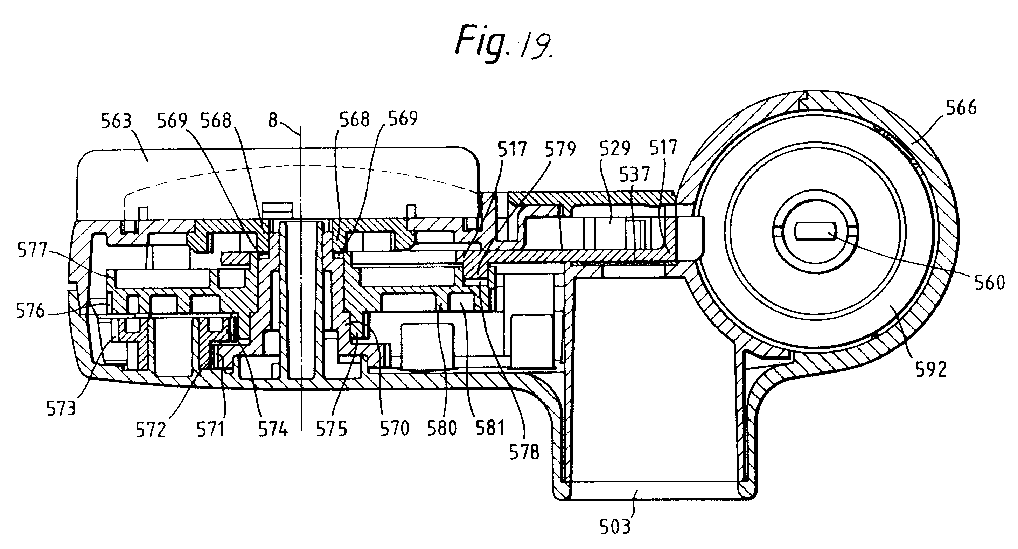

When the operator wishes to use the inhaler he pushes the slider (563) diametrically across the wheel (564) to its full extent, overcoming detents formed by a rib (567a) on the slider (563) which rides over a rib (567b) on the wheel (564), and rotates the wheel and slider assembly clockwise until the slider hits a stop formed by the rear cover (565) resulting in a limit of 180° of movement about vertical axis (8). Formed as part of the wheel (564) are drive dogs (568) which, as shown in Fig. 19, protrude into the inhaler through the rear cover (565). These drive dogs (568) locate precisely with recesses (569) in a spindle (570) and cause the spindle (570) also to rotate clockwise through 180°. Integral with this spindle (570) is a gear (571) meshing with a gear (572) which forms one part of a compound gear unit (573). The second gear (574) of the compound gear unit (573) meshes with a gear (575) which is part of a cam plate (576).

The cam plate (576) comprises three functional surfaces (577, 580 and 587).

The first functional surface (577) in the upper face of the cam plate (576) has a continuous cam track recess (578) which when rotated transfers linear movement to the captive cam follower (579) attached to the ejector plate (517) causing the ejector plate (517) to move forwards and backwards across the swirling chamber (529).

The second functional surface (580) in the underside of the cam plate also has a continuous cam track recess (581) which when rotated transfers movement to a captive cam follower peg (582) (Fig. 18) attached to a rocker arm (583). When actuated the rocker arm (583) oscillates by symmetrically pivoting about its centre; it has attached to each end one of two diametrically opposite pegs (582) and (584). The peg (584) fits in a yoke (585) which forms part of the indexing rod (586). Thus, when the cam follower peg (582) is caused to move, the rotation of rocker arm (583) is transferred to linear action of the indexing rod (586). The indexing rod (586) has attached to its end a claw (521) for causing the rotary indexing of the magazine drive mechanism.

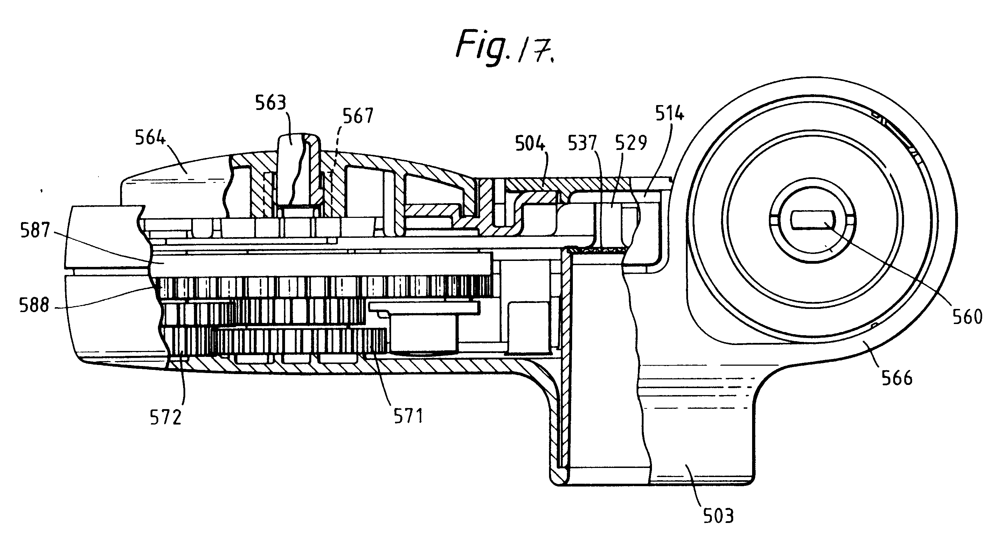

The third, circumferential, functional surface (587) (Fig. 17) of the cam plate (576) (Fig. 19) has for the majority of its length an even pattern of relief (588) which when acted upon by an integrally sprung pawl (589) (Fig. 18) creates an audible sound as confirmation of operation throughout the length of the circumferential surfaces (587), the pattern of relief (588) is interrupted by a number of local recesses (590) each of which has a shape compatible with the nose of the pawl (589) such as to prevent reverse rotation of the cam plate (576) once the ratchet nose is in the recess (590). The degree of rotation of the cam plate (576) relative to the rotation of the slider (563) is a function of the gear ratio selected and may be chosen by suitable gear ratios found to be advantageous in use.

FIGURES 20 and 21 show the end cap (561) at the opposite end to the magazine loading aperture (562). The end cap (561) has mounted on it a numbering wheel (591) which is driven by an indexing wheel (592) through castellated drive dogs (593). The indexing wheel (592) is driven by engagement with the claw (521) of the reciprocable indexing rod (586). Axial movement of the indexing wheel (592), against the compression spring (594), is allowed by virtue of the castellated drive dogs (593).

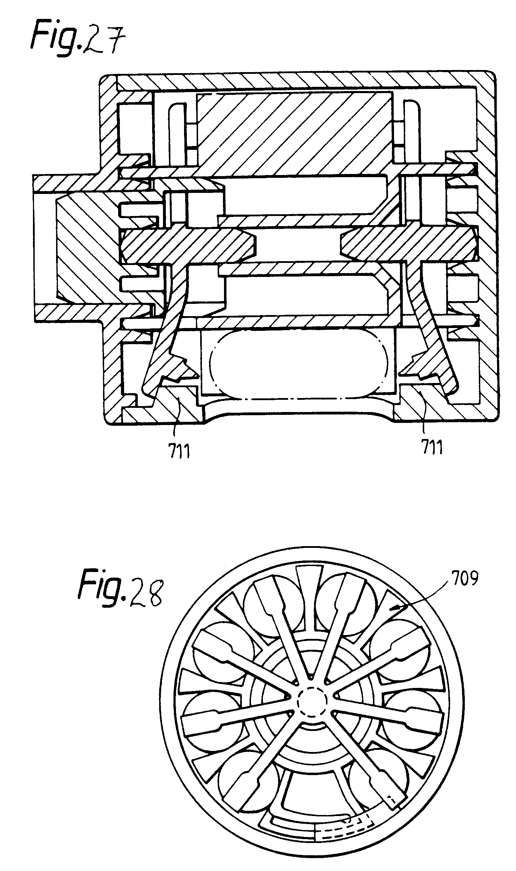

The magazine (623 shown in Figs. 23 to 25,or 723 shown in Figs. 26 to 28) is loaded through an aperture (562) of the inhaler front cover (566) with the drive blade (614, 714) on the magazine innermost. The drive blade (614 or 714) locates in the slot of the indexing wheel (595) at which time resistance will be felt as the magazine depresses the indexing wheel against its compression spring (594). The final travel of loading the magazine causes it to be retained by a catch detail created by a dimple (600) on the outer casing (636) (Fig. 23) of the magazine and an internal wall on the front cover which supports an integrally moulded spring strap (596) (Fig. 15). As the magazine (623 or 723) is inserted, the indexing wheel (592) is pressed against the compression spring (594) to a position which allows the forward motion of the claw (521) on the indexing rod (586) (Fig. 18) to rotate the indexing wheel (592) in an anticlockwise direction resulting in the central hub (631 shown in Fig. 23, or 709 in Fig. 25) of the magazine to be indexed rotationally to the next capsule station.

During the indexing stages of the magazine, a cam follower (597) (Fig. 20) mounted in the end cap (561) travels perpendicular to the axis of the end cap within a 360° are involute cam recess (598) on the numbering wheel (591). This involute cam recess has stopped ends which act as limiters and restrict the travel to one revolution of the central hub of the magazine (631) (Fig. 23) to avoid re-use of capsules. Provided the capsule ejector plate is withdrawn from the magazine the magazine ejector button (560) can be depressed, thereby overcoming the spring strap (596), to eject the spent magazine. Preferably this is effected after one revolution of the magazine, corresponding to use of all eight capsules. The rewind torsion spring (599) can then return the numbering wheel to the initial position ready for reloading with a new magazine, because the claw (521) no longer engages the indexing wheel (592).

A third embodiment of a rotary magazine, to be used with the inhaler of the second embodiment is now described with reference to Figures 23 to 25.

Figures 23 to 25 show a rotary magazine (623) formed with two main parts, a stationary part consisting of end cap (633) and outer casing (636) to be held in place when loaded in an inhaler and a rotor consisting of two end hubs (632) integrally fixed to a central hub (631).

The capsules, eight of them in the drawings, are held in recesses (625) in the periphery of the rotor central hub (631) with the longitudinal axis of the capsules and recesses parallel to the axis of rotation of the rotor assembly (631, 632). The rotor comprises the central hub (631) between two end hubs (632) fixed thereto. In Figure 24 it can be seen that the capsules (25) are maintained in position by tips (601) of pins (605).

In this third embodiment of magazine the capsules are already pierced during or prior to loading in the magazine. The pins (601), as well as maintaining the capsules in position in their recesses, also serve to plug the holes formed in the ends of the capsules. These holes can exist either before the capsules are loaded into the rotary magazine (623) or may be made as the rotary magazine (623) is assembled or loaded.

Figure 24 is an enlarged view from Figure 23 and shows the plug, or tip (605) of a pin (601), maintained within the ends of a capsule. The sealing means (609), for example a fillet radius (606) at the base of the pin (601), are formed so as to maintain a seal around the ends of the capsules, to prevent escape of powder and, possibly more importantly, to prevent air from entering an unused capsule to contaminate it or to allow its contents to degrade. In this fashion the integrity of the capsules is maintained although they are already pierced.

Additionally, it is envisaged that when fresh the magazine could be sealed into suitable outer packaging such as a sachet or blister pack to provide further protection against the environment prior to insertion into the inhaler.

As in the case of Figure 11a there is a blank position (621) in the rotary magazine. The blank position, which may be an empty recess, prevents the waste of a capsule which would otherwise be released immediately upon insertion of the magazine.

Formed within the third type of rotary magazine (623) is a pair of capsule release cam tracks (617), one track being situated towards each end of the magazine. At the open end which is the drive end of the magazine these tracks are defined by a wall (637) of the outer casing and track pieces (638) (Fig. 23) integrally mounted in the outer casing, and at the closed or non-drive end they are defined by track pieces (638). The cam tracks (617), the track pieces (638), and the end cap (633) are stationary parts and may be formed integrally with or separate from the outer casing (636), depending upon the method of assembly of the magazine. The locus of the tracks is the same as that shown in Figure 9.

The piercing pins (601) are formed with pegs (619) as followers to fit into the release tacks (617) between the track pieces. As the rotor comprising end hubs (632) and central hub (631) rotates and the pins (601) rotate with them, so the pegs (619) of the pins (601) follow the tracks (617). The pins are formed to be axially movable within the rotor comprising end hubs (632) and central hub (631).

As the drive pegs (619) of the pins follow the tracks (617) they are caused, at the bulges, to move out towards the ends of the magazine. The bulges are situated at the 'use position', i.e. where a recess in a magazine inserted into the inhaler is part of the swirling chamber (529) of the inhaler and a capsule in that recess is to be used. At this point, therefore, the two piercing pins (601) holding that capsule move apart, thereby unplugging their capsule (25) and releasing it. Thus, the capsules are plugged tightly until their recesses reach this 'use position' where they are released. When, after use, they are returned to their recesses by the ejector plate (517) of the inhaler the capsules are kept in their recesses as the magazine is rotated. As a recess is rotated away from the 'use position', the drive pegs of the pins (601) of that recess continue to follow the cam tracks (617); they come towards each other again, and re-plug the used capsule. This is useful, not only in preventing capsules from moving about freely, possibly jamming the magazine, but also in preventing any residual powder in the spent capsules from escaping to contaminate the inhaler, and causing it to need further cleaning.

In the above described third magazine embodiment although the capsules may be pierced before loading into the magazine, the pins are so shaped that they can, if desired, pierce the capsules upon loading of the magazine with the capsules. Alternatively they can be shaped so that they merely provide a plug for pre-pierced capsules.

A fourth embodiment of a rotary magazine, to be used with the inhaler of the second embodiment, is shown in Figures 26 to 28. This magazine operates to a similar manner to the magazine of the second embodiment shown in Figures 12 to 14.

The casing (700) has raised portions (711) at both ends of the capsule opening. As the drive block (714) is rotated, it rotates the central hub (709) and two daisy wheels (713). As the ends (703) of the arms (701) of the daisy wheels (713) pass by the raised portions (711) they are bent outwards, so releasing the capsule held between them by plugs (702) on those arms.

Although in the described magazines the mechanism which causes a capsule to be released, that is the cam tracks and protrusions, is part of the magazine itself, this does not have to be the case. To simplify the magazines, there may be tracks or protrusions associated with, or in, the walls of the magazine chamber (39, 339, 539) which cause the capsules to be released at the proper 'use position'. Similarly the magazines could be provided with piercing pins which are guided by tracks in the magazine chamber walls to pierce a capsule just before use. Such tracks or protrusions in the walls of the magazine chamber could lead to simplified rotary magazines and prevent the need for a new magazine to be inserted in a particular position rather than just in a particular orientation.

The present invention may be used with many types of inhalation mechanism whereby the contents of a capsule are extracted or drawn out. However, it is preferable that it should be used in a system where the contents of a capsule are drawn out via holes formed or pierced in the capsule, preferably in its ends. More particularly the contents are extracted from a capsule by a combination of pneumatic action, centrifugal action, and impact of a pierced capsule with the lateral wall of the swirling chamber.

The inhalation system is described with reference to all of the above-described inhaler embodiments. There is provided a swirling chamber (29, 329, 529) having a substantially circular peripheral side wall (30, 330, 530) adjoining flat end walls defined by a mesh (37, 337, 537) and a wall. There is a chordal recess (41, 341, 541) in the side wall which is part of the rotary magazine (23, 323, 623, 723) which forms one part of the chamber (29,329). It is from this recess that a capsule is presented, tangentially to the circular peripheral walls, and to which the capsule will return after use.

The chamber (29, 329, 529) furthermore communicates with two air inlets (14, 314, 514) which clearly generate a swirling motion in the chamber about an axis which is generally central of this chamber and extends perpendicular to the plane of the paper in Figures 4, 17 and 28, as air is aspirated through the mouthpiece nozzle (3, 303, 503). There is a direct passage between the chamber (29, 329, 529) and the mouthpiece (3, 303, 503) in which the only barrier is the mesh (37, 337, 537). Since, in the present invention the capsule will remain in one piece and, at least in the first inhaler embodiment, there should be no other fragments, it is not necessary that the mesh should be particularly fine (which could possibly result in it trapping particles of the contents of the capsule) but instead it may be quite coarse, needing only to prevent the capsule itself from leaving the swirling chamber (29, 329, 529) whilst allowing the capsule to rotate around the chamber without being trapped. This reduces the air flow throttling effect of the mesh on the inhalation stream.

Once a fresh capsule is presented within the swirling chamber (29, 329, 529) through the use of the recharging mechanism, the operator simply inhales through the mouthpiece to generate the necessary swirling air stream into the swirling chamber (29, 329, 529) through the inlets (14, 314, 514). This same swirling action will, if necessary, detach the capsule from the recess (41, 341, 541) in the use position and will cause the capsule to rotate rapidly about the above-mentioned axis of rotation of the vortical swirling air flow in the chamber. The capsule is to be of a length shorter than the diameter of the chamber and this means that it is able to be spun round its transverse axis in the vortical air flow. At the same time the length of the capsule may allow it to contact the peripheral wall of the chamber so as to sustain impacts which help remove the contents, that is the pulverulent medicament, from within the capsule by percussive action.

This degree of impact with the walls of the chamber is enhanced by the presence of the recess (41, 341, 541) giving the chamber a generally non-symmetrical or eccentric appearance, resulting in random and rapidly occurring impacts which augment any centrifugal emptying of the spinning capsule shell. Further impacts may be sustained if the end tab of the ejector plate protrudes slightly into the chamber or is recessed slightly, either case forming further impact surfaces. When inhalation is complete, the medicament will almost completely have been emptied from the capsule and indeed also from the chamber by being exhausted through the mesh.

Provided the inhaler is kept dry, and if the materials chosen for all appropriate parts, e.g. medicament-contacting parts, of the inhaler in accordance with the present invention are ones which have a relatively low electrostatic attraction for the contents of the capsule, the inhaler will not need regular cleaning when used by the same operator. The mesh may be of a material which is electrically conductive or is otherwise antistatic. Preferably it may be of a conductive polymer though it may be of a metal such as stainless steel.

With regard to all embodiments discussed above, the exact positioning of the magazine chambers, magazines, swirling chamber, mouthpiece etc. relative to each other is not important and may be varied in the inhaler.

The actual shape of the swirling chamber may also be varied in accordance with the present invention provided the capsule-emptying action is still achieved.

The magazines, which are loaded in the factory, may be assembled by clipping together or preferably by welding. If a magazine is welded it helps prevent possible tampering which, since this inhaler is for the provision of medicaments, could have very dangerous consequences.

It is possible that the inhalers of the present invention can be as automated and made as 'user friendly' as possible. The mechanisms such as rotating the magazine to load a capsule, emptying the swirling chamber, etc. can be motorised and it is envisaged that logic circuits can be introduced whereby a magazine is prevented from going round more than once. Other features which such a logic system could include would be to provide a visual and/or audible alarm to indicate when the seventh or penultimate capsule or eighth or last capsule, of a magazine has been used, thereby helping to prevent someone from finding out that he has run out of capsules when he is most in need of them. Further features might include that the inhaler does not work once all the capsules have been used or possibly that the magazine chamber cover will click open when the last capsule has been used, or even that the magazine can only be removed when it is in the orientation of just having been inserted, or of having had all its capsules used if this is different.

Not all these features need to be worked through a logic circuit. Whether or not such logic circuits are included, an indicator could be worked mechanically whereby the user can be readily made aware how many unused capsules remain, for example through the use of a window on the main body which shows an increasing amount of red as the capsules in the magazine are used up. Possibly there may be a keyway, or some other mechanical means whereby the magazine chamber cover may not be opened, or a magazine may not otherwise be removed when it is not in its start or finish position, that is the position it is in when it is inserted, (or the position it is in when all the capsules have been used, if this is different).

It is possible that in some of these embodiments the last capsule may be used and still left in the swirling chamber as the magazine is changed. The chamber may be provided with a removable cover for removing the last capsule, which cover could also be used for manual charging of the swirling chamber. Alternatively, or additionally, there may be provided mechanical or electrical means whereby a magazine cannot be changed until the last capsule has been ejected. Or, if the magazines have an empty recess in their blank positions, the last used capsule in a previous magazine can be placed in the empty recess of the new magazine.

If capsules are pre-pierced before loading into the magazine this produces less risk of small pieces of capsule likely to cause problems in the user's air tract as might happen in the case of an asthmatic. It is envisaged that capsules could be pre-pierced using a laser to make holes in each end before the capsules are loaded into a magazine and sealed therein. With such capsules, and others where there is no danger from fragments of capsule, it might be possible to substitute the mesh in the swirling chamber by some form of a mixer to mix the capsule contents with the air, provided such a mixer preserves the chamber geometry and retains the capsule.

It is possible for devices according to the present invention to be provided with spare magazine or capsule chambers so that an inhaler user may always have a good supply of capsules with him. Another addition might be a mouthpiece cover pivoted or otherwise attached to the inhaler, provided to improve hygiene.

The method by which the capsules are loaded into the magazines is not particularly important, though it is preferable that in the case of some of these described magazines they are provided in an orientation whereby the blank position may be positioned in the 'use position' initially. It is envisaged that magazines could be re-used.

## Claims

1. A rotary magazine (23) for use in an inhaler, comprising a plurality of recesses (125, 215) for housing capsules (25), and means (101, 201, 203), in a plurality of said recesses (125, 215), for holding the capsules (25); characterised
in that said magazine (23) is adapted to receive pierced capsules (25);
by further comprising means (105, 109, 202) for plugging pierced holes in the capsules (25), and means (117, 119, 205, 211) to release said capsules (25) one at a time, from said magazine (23); and
in that said plugging means (105, 109, 202) are provided in a plurality of individual ones of said recesses.

2. A rotary magazine (23) according to claim 1, wherein said holding means (101, 201, 203) and said plugging means (105, 109, 202) are integral.

3. A rotary magazine (23) according to claim 1 or 2, wherein said holding means (101, 201, 203) and said plugging means (105, 109, 202) are provided at both ends of the recesses (125, 215).

4. A rotary magazine (23) according to claim 1, 2 or 3, wherein said plugging means (105, 109) comprise piercing means (105) for piercing said capsules (25).

5. A rotary magazine (23) according to any one of the preceding claims, further comprising capsules (25) loaded in said recesses (125, 215).

6. A rotary magazine (23) according to claim 1, 2 or 3 further comprising capsules loaded in said recesses (215) and provided with holes before they are loaded.

7. A rotary magazine (23) according to any one of the preceding claims, wherein the recesses may be rotated (125, 215), one by one, to a use position within said magazine (23).

8. A rotary magazine (23) according to claim 7 further comprising release tracks (117), wherein said capsule holding means (101) further comprise track followers (119) which follow said tracks (117) as the recesses (125) are rotated, and wherein at said use position of said magazine (23) the tracks are contoured (123) whereby the followers (119) move apart for releasing capsules (25) held by said holding means (101) at said use position.

9. A rotary magazine (23) according to claim 7 further comprising catching means (211) provided in the magazine (23) at said use position, and raised portions (205) formed on said holding means (201), wherein when a recess (215) is rotated to the use position the raised portions (205) on said holding means (201) are rotated to the use position and are caught on the catching means (211), whereby they are moved apart for releasing a capsule (23) held by the holding means (201,203) at said use position.

10. A rotary magazine (23) according to any one of the preceding claims, wherein the plugging means (105, 109, 202) further comprise sealing means (109, 202) for sealing pierced holes against the environment.

11. A rotary magazine according to any one of the preceding claims, further comprising means (13) which indicate the number of used or unused capsules (25).

12. An inhaler in combination with a magazine (23) according to any one of the preceding claims.

## Patentansprüche

1. Rotationsmagazin (23) zur Verwendung in einem Inhalator, mit einer Mehrzahl von Vertiefungen (125, 215) zur Aufnahme von Kapseln (25), und mit Mitteln (101, 201, 203), in einer Mehrzahl der Vertiefungen (125, 215), zum Halten der Kapseln (25); dadurch gekennzeichnet,
daß das Magazin (23) eingerichtet ist, aufgestochene Kapseln (25) aufzunehmen;
daß es ferner Mittel (105, 109, 202) zum Verschließen aufgestochener Löcher in den Kapseln (25) und Mittel (117, 119, 205, 211) zur Freigabe der Kapseln (25), jeweils einzeln, aus dem Magazin (23) aufweist; und
daß die Verschließmittel (105, 109, 202) in einer Mehrzahl einzelner der Vertiefungen vorgesehen sind.

2. Rotationsmagazin (23) nach Anspruch 1, bei welchem die Haltemittel (101, 201, 203) und die Verschließmittel (105, 109, 202) einteilig sind.

3. Rotationsmagazin (23) nach Anspruch 1 oder 2, bei welchem die Haltemittel (101, 201, 203) und die Verschließmittel (105, 109, 202) an beiden Enden der Vertiefungen (125, 215) vorgesehen sind.

4. Rotationsmagazin (23) nach Anspruch 1, 2 oder 3, bei welchem die Verschließmittel (105, 109) Aufstechmittel (105) zum Aufstechen der Kapseln (25) umfassen.

5. Rotationsmagazin (23) nach einem der vorhergehenden Ansprüche, ferner mit Kapseln (25), die in den Vertiefungen (125, 215) geladen sind.

6. Rotationsmagazin (23) nach Anspruch 1, 2 oder 3, ferner mit Kapseln (25), die in den Vertiefungen (125, 215) geladen und mit Löchern versehen sind, bevor sie geladen werden.

7. Rotationsmagazin (23) nach einem der vorhergehenden Ansprüche, bei welchem die Vertiefungen (125, 215) eine nach der anderen in eine Verwendungsposition innerhalb des Magazins (23) gedreht werden können.

8. Rotationsmagazin (23) nach Anspruch 7, ferner mit Freigabebahnen (117), wobei die Kapselhaltemittel (101) weiters Bahnstößel (119) aufweisen, die den Bahnen (117) bei der Rotation der Vertiefungen (125) folgen, und wobei in der Verwendungsposition des Magazins (23) die Bahnen mit Konturen (123) versehen sind, wodurch sich die Stößel (119) zur Freigabe von Kapseln (25), die durch die Haltemittel (101) in der Verwendungsposition gehalten werden, auseinander bewegen.

9. Rotationsmagazin (23) nach Anspruch 7, ferner mit Eingriffsmitteln (211), die im Magazin (23) in der Verwendungsposition vorgesehen sind, und erhöhten Teilen (205), die an den Haltemitteln (201) gebildet sind, wobei, wenn eine Vertiefung (215) in die Verwendungsposition gedreht wird, die erhöhten Teile (205) an den Haltemitteln (201) in die Verwendungsposition gedreht werden und an den Eingriffsmitteln (211) erfaßt werden, wodurch sie zur Freigabe einer Kapsel (25), die von den Haltemitteln (201, 203) in der Verwendungsposition gehalten wird, auseinander bewegt werden.

10. Rotationsmagazin (23) nach einem der vorhergehenden Ansprüche, wobei die Verschließmittel (105, 109, 202) ferner Abdichtmittel (109, 202) zum dichten Verschließen aufgestochener Löcher gegen die Umgebung aufweisen.

11. Rotationsmagazin nach einem der vorhergehenden Ansprüche, ferner mit Mitteln (13), die die Anzahl verwendeter oder nichtverwendeter Kapseln (25) anzeigen.

12. Inhalator in Kombination mit einem Magazin (23) nach einem der vorhergehenden Ansprüche.

## Revendications

1. Magasin rotatif (23) propre à être utilisé dans un inhalateur, comprenant une pluralité de cavités (125, 215) destinées à contenir des capsules (25), et des moyens (101, 201, 203), dans une pluralité desdites cavités (125, 215), pour retenir les capsules (25);
caractérisé en ce que ledit magasin (23) est à même de recevoir des capsules percées (25);
du fait qu'il comprend en outre des moyens (105, 109, 202) destinés à boucher des trous percés dans les capsules (25), et des moyens (117, 119, 205, 211) pour libérer lesdites capsules (25) une à la fois, dudit magasin (23), et
en ce que lesdits moyens de bouchage (105, 109, 202) sont prévus dans une pluralité de cavités individuelles desdites cavités.

2. Magasin rotatif (23) suivant la revendication 1, dans lequel lesdits moyens de retenue (101, 201, 203) et lesdits moyens de bouchage (105, 109, 202) sont venus d'une seule pièce.

3. Magasin rotatif (23) suivant la revendication 1 ou 2, dans lequel lesdits moyens de retenue (101, 201, 203) et lesdits moyens de bouchage (105, 109, 202) sont prévus aux deux extrémités des cavités (125, 215).

4. Magasin rotatif (23) suivant la revendication 1, 2 ou 3, dans lequel lesdits moyens de bouchage (105, 109) comprennent des moyens de perçage (105) pour percer lesdites capsules (25).

5. Magasin rotatif (23) suivant l'une quelconque des revendications précédentes, comprenant en outre des capsules (25) chargées dans lesdites cavités (125, 215).

6. Magasin rotatif (23) suivant la revendication 1, 2 ou 3, comprenant en outre des capsules chargées dans desdites cavités (215) et pourvues de trous avant d'être chargées.

7. Magasin rotatif (23) suivant l'une quelconque des revendications précédentes, dans lequel les cavités peuvent être tournées (125, 215), une à une, dans une position d'utilisation dans ledit magasin (23).

8. Magasin rotatif (23) suivant la revendication 7, comprenant en outre des chemins de came de libération (117), dans lequel lesdits moyens de retenue de capsules (101) comprennent en outre des suiveurs (119) qui suivent lesdits chemins de came (117) à mesure que les cavités (125) tournent, et dans lequel, dans ladite position d'utilisation dudit magasin (23), les chemins de came sont modelés (123), grâce à quoi les suiveurs (119) s'écartent l'un de l'autre pour libérer des capsules (25) retenues par lesdits moyens de retenue (101) dans ladite position d'utilisation.

9. Magasin rotatif (23) suivant la revendication 7, comprenant en outre des moyens d'arrêtage (211) prévus dans le magasin (23) dans ladite position d'utilisation, et des parties surélevées (205) formées sur lesdits moyens de retenue (201), dans lequel, lorsqu'une cavité (215) est amenée par rotation dans la position d'utilisation, les parties surélevées (205) sur lesdits moyens de retenue (201) sont amenées par rotation dans la position d'utilisation et sont arrêtées par les moyens d'arrêtage (211), grâce à quoi ils sont écartés pour libérer une capsule (25) retenue par les moyens de retenue (201, 203) dans ladite position d'utilisation.

10. Magasin rotatif (23) suivant l'une quelconque des revendications précédentes, dans lequel les moyens de bouchage (105, 109, 202) comprennent en outre des moyens d'étanchéité (109, 202) pour protéger des trous percés contre l'environnement.

11. Magasin rotatif suivant l'une quelconque des revendications précédentes, comprenant en outre des moyens (13) qui indiquent le nombre de capsules utilisées ou non utilisées (23).

12. Inhalateur en combinaison avec un magasin (23) suivant l'une quelconque des revendications précédentes.
